# EUROPEAN PATENT APPLICATION

(11) **EP 4 821 922 A1**
(43) Date of publication of application: **14.10.2026**
(21) Application number: 25184458.5
(22) Date of filing: 23.06.2025
(51) Int. Cl.: C07D 251/60, C07D 251/62, B01J 19/00

(54) **PROCESS FOR THE PRODUCTION OF HIGH-PURITY MELAMINE FROM THE PYROLYSIS OF UREA, WITH OPTIMIZATION OF THE DEAMMONIATION STEP OF THE MOTHER LIQUORS**

(30) Priority: 07.04.2025 IT 202500007449
(71) Applicant: EUROTECNICA CONTRACTORS AND ENGINEERS S.p.A., 20159 Milano (IT)
(72) Inventor: DE AMICIS, Alberto, I-20097 San Donato Milanese (MI) (IT); SANTUCCI, Roberto, I - 21050 Gorla Maggiore (VA) (IT); SERRAFERO, Alberto Carlo, 20098 San Giuli San Giuliano Milanese (MI) (IT); PETRILLO, Francesca, 20161 Milano (IT); RABAIOLI, Matteo, 24040 Casirate D adda (BG), (IT)
(74) Representative: Bird & Bird Società tra Avvocati S.r.l.

(57) **Abstract**

A process is described for the production of high-purity melamine from the pyrolysis of urea, with optimization of the deammoniation step of the mother liquors.

## Description

The present invention relates to a process for the production of high-purity melamine by the high-pressure pyrolysis of urea, with optimization of the deammoniation step of the mother liquors.

More specifically, the invention relates to a process that involves the purification in aqueous ammonia solution of the melamine produced in the pyrolysis reactor, and its separation by crystallization.

It is known that the transformation of molten urea into melamine is described by the following overall reaction (1):

6 NH₂CONH₂ → (CN)₃(NH₂)₃ + 6 NH₃ + 3 CO₂ (1)

urea melamine ammonia carbon dioxide according to which for every kilogram of melamine, 1.86 kilograms of NH₃ and CO₂ are formed, globally called off-gases.

One of the most widespread industrial processes, based on the high-pressure pyrolysis of urea is that described in US patent 3,161,638 in the name of Allied. The process described in the above-mentioned patent is jeopardized by a high consumption of raw materials and utilities (such as steam, cooling water, fuel gas, electricity) and is penalized by an inefficient integration with the urea plant upstream due to the characteristics of the streams recycled to the same. The solution to some of the drawbacks of this process is described in the state of the art represented by US patents 7,125,992 and EP 2385043 (corresponding to ITMI201000810), a solution that provides a reduction in energy consumption, investment and specific consumption of urea necessary for the production of melamine.

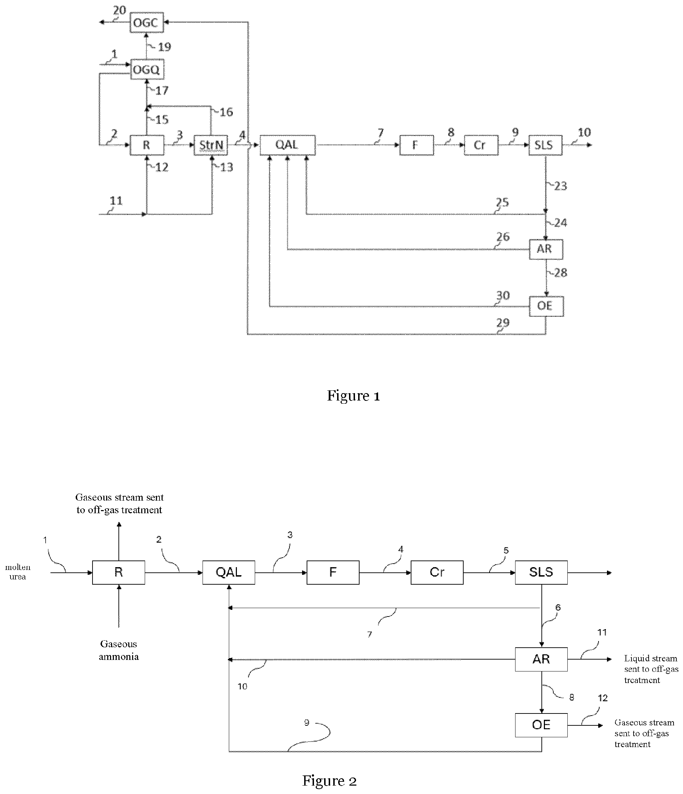

In a subsequent patent application EP4245755, a process for the production of melamine was identified which allows for a substantially economic recovery and recycling of the ammonia and process water contained in the mother liquors and which allows for the recycling of one or more streams containing CO₂ and NH₃, with no or a low water content, in the urea synthesis plant by means of the following steps (represented in figure 1):
the product of the urea pyrolysis reaction, carried out at a temperature ranging from 360 to 450°C and at a pressure higher than 7.0 MPa, consisting of a gaseous phase (15) and a liquid phase (3), is subjected to subsequent treatments, wherein:
- the liquid phase (3) exiting from the Pyrolysis Reactor (R) containing melamine, unreacted urea, oxidized intermediate pyrolysis products (OATs) and products of the deammoniation and condensation of melamine (polycondensates), is sent to a subsequent reaction step in a reactor or Post-Reactor (StrN) that operates under temperature and pressure conditions substantially equal to those of the Pyrolysis Reactor (R) and into which a stream of gaseous, anhydrous and superheated NH₃ under pressure is fed (13), to eliminate the dissolved CO₂, complete the urea pyrolysis reaction and transform most of the OATs into melamine, obtaining a liquid phase of melamine (4) and a second gas phase (16);
- the anhydrous gaseous phase from the Pyrolysis Reactor (15) and the anhydrous gaseous phase from the Post-Reactor (16) are combined and the off-gas stream thus obtained (17) is subjected to a washing (OGQ) with molten urea (1) to recover the melamine contained therein as steam, before said washed off-gas stream (19) is sent back to a urea synthesis process to recover the NH₃ and CO₂ contained therein;
- the liquid phase exiting from the Post-Reactor (4) is brought in aqueous solution to a quench-ammonolysis section (QAL), in the presence of NH₃, keeping the whole mixture under controlled conditions of temperature and residence time preferably at a temperature ranging from 160 to 180°C, more preferably from 170 to 172°C, at a pressure ranging from 2.5 to 4.5 MPa, more preferably equal to 2.5 MPa, for a time ranging from 30 to 60 minutes, more preferably 45 minutes, obtaining a solution substantially free of polycondensates (7) which is subjected to crystallization (Cr) to give high-purity melamine (10) and mother liquors (23) containing melamine and reduced quantities of OATs, a part of said mother liquors (25) being recycled, without any treatment, to the quench phase in the quench-ammonolysis section (QAL);
- the remaining part of the mother liquor (24) not recycled to the quench-ammonolysis section (QAL) is treated in a deammoniation section (AR) from which a stream containing water and ammonia (26) is obtained by distillation, which is in turn sent to the quench-ammonolysis section (QAL) and a stream (28) containing water, CO₂ and OATs, practically free of NH₃, which is sent to the subsequent elimination phase of the OATs in the OATs elimination section (OE);
- said OATs elimination step produces a liquid phase (30) substantially composed of water which is recycled to the quench-ammonolysis section (QAL) and a gaseous phase (29), comprising NH₃ and CO₂ saturated with water, coming from the OATs elimination section (OE) wherein said gaseous phase (29)
   - is combined with the washed off-gas stream (19) before being sent back (20) to a urea synthesis process for the recovery of the NH₃ and CO₂ contained therein, or
   - is recycled to the deammoniation section (AR), obtaining a liquid phase (31) containing NH₃, CO₂ and water, which is combined with the washed off-gas stream (19) before being sent back (20) to a urea synthesis process for the recovery of the NH₃ and CO₂ contained therein, or
   - is recycled to the deammoniation section (AR), obtaining a liquid phase (31) containing NH₃, CO₂ and water, which is sent back to a urea synthesis process for the recovery of the NH₃ and CO₂ contained therein.

It has been found, however, that in the process described in EP'755, during the functioning of the distillation column for the deammoniation or recovery of ammonia from mother liquors under the process conditions described (temperature at the bottom of the column equal to 205°C and pressure equal to 1.6 Mpa), a part of the ammonia recovery section AR, i.e. the distillation column itself, undergoes significant fouling. In particular, the efficiency of the lower plates of the distillation column decreases substantially in just two months from the start-up of the AR ammonia recovery section. This fouling of the distillation column in such a short time must therefore be taken into account already in the design of the column itself, with the result that, due to the reduced efficiency of the lower plates, the number of plates and their size must be increased compared to what would be expected in a distillation column that does not include this fouling effect. This clearly entails higher investment costs for both the column and for its auxiliary equipment and instruments. Furthermore, the maintenance of the interior of the column requires frequent inspections and maintenance due to the low efficiency of the column plates themselves.

The fouling of the distillation column has the undesirable effect of a continuous adjustment of the operating parameters of the column by the plant operators. The loss of efficiency of the column therefore leads over time to a modification of the flow-rate of the streams fed, and specifically leads to a reduction in the main supply stream of mother liquors which forces the operator to reduce the plant load and therefore with a lower production of melamine.

The objective of the present invention is therefore to identify a process for the production of melamine that overcomes the drawbacks previously indicated.

The present invention therefore relates to a process for the production of high-purity melamine with high yields by the pyrolysis of urea carried out at a temperature ranging from 360 to 450°C and at a pressure higher than 7.0 MPa, comprising a deammoniation step or recovery of ammonia from a mother liquor stream coming from a liquid/solid separation step SLS of an aqueous ammonia stream containing melamine crystals, wherein said mother liquor stream 6 is treated in a deammoniation section AR from which the following are obtained by distillation:
~ a stream 10, composed of NH₃ and water, which is recycled to the quench-ammonolysis section QAL;
~ a stream 11, composed of water, CO₂ and NH₃ which is sent to an off-gas treatment step or recycled directly into the urea plant for the recovery of NH₃ and CO₂;
~ a stream 8, containing water, OATs and a quantity of NH₃ that ranges from 0.1 to 2% by weight with respect to the weight of stream 8, which is sent to the subsequent section (OE) for the elimination of the OATs,
said distillation step being effected in a distillation column with a temperature at the bottom of the column not exceeding 185°C, preferably ranging from 150 to 180°C, and even more preferably from 115 to 150°C.

Said distillation step is preferably carried out at a pressure of 0.2 to 1.0 MPa.

The process according to the present invention is represented in the block diagram of figure 2.

It has in fact been surprisingly found that operating the column at temperatures lower than those indicated in the state of the art reduces the formation of new OATs by hydrolysis of the melamine contained in the solution sent to the column (the hydrolysis reaction of melamine is unfavourable at low temperatures) and at the same time a minimal increase in the concentration of ammonia at the bottom of the column counteracts the precipitation of OATs already present, effectively preventing fouling of the lower plates. Specifically: reducing the temperature at the bottom of the distillation column from 205°C to 195°C extends the efficiency of the plates for up to about three months, whereas lowering the temperature at the bottom of the column to a temperature not higher than 185°C has allowed the fouling of the lower plates to be completely eliminated. A limit temperature has therefore been surprisingly found below which fouling/encrustment of the column plates is no longer observed.

The identification of an optimal temperature at the bottom of the column, lower than that described in the state of the art, is surprising for a skilled person in the field, who operates the bottom of the distillation column at temperatures that are generally higher than that defined by the present invention, in order to obtain the complete removal of NH₃. A skilled person in the field knows that, as the temperature at the bottom of the column increases, the separation of ammonia from the mother liquors is more efficient and this allows a stream exiting from the bottom of the column substantially free of NH₃, to be obtained. A skilled person in the field knows that as the plates lose their efficiency, the bottom of the column will tend to have a higher concentration of NH₃; therefore, in order to guarantee a stream from the bottom substantially free of ammonia, the flow of steam on the bottom of the column reboiler must be increased in order to maintain the temperature at the equilibrium point PT (Pressure - Temperature) of the water solution free of NH₃. Maintaining a high bottom temperature to compensate for the loss of efficiency and having a solution free of NH₃, facilitating the precipitation of OATs, increases the fouling rate of the plates, also leading to excessive and unnecessary steam consumption.

In order to avoid the precipitation of OATs on a bottom free of NH₃, in order to increase their solubility, a skilled person in the field would tend to increase the temperature at the bottom by increasing the pressure of the column and thus increasing the phenomenon of the formation of OATs by hydrolysis..

It has been surprisingly found by the applicant that, by operating the column from its start-up at a temperature not exceeding 185°C and at lower pressures, with a quantity of NH₃ at the bottom which is such as to avoid the precipitation of OATs, the establishment of the vicious circle described above can be avoided. Despite the lower temperature, it has been surprisingly found that, being in the absence of CO₂, a small concentration of ammonia at the bottom is sufficient to ensure a pH which is such as to keep the OATs in solution avoiding their possible precipitation even at low temperatures and pressures. This results in a great economic advantage, as it will be possible to use a reboiler steam having a lower quality (i.e. lower pressure and temperature) and less expensive equipment.

The process according to the present invention is represented in the attached figure 2. The block diagram of figure 2 substantially shows the main sections and flow lines of the process according to the present invention.

### Deammoniation section (AR):

In the process according to the present invention, the part of the mother liquor (stream 6) coming from the solid/liquid separation section, not recycled to the quench-ammonolysis section (QAL), is treated in the deammoniation section (AR), in order to obtain:
~ a stream 10, composed of NH₃ and water, which is recycled to the quench-ammonolysis section (QAL)
~ a stream 11, composed of water, CO₂ and NH₃, which is sent to an off-gas treatment step or recycled directly into the urea plant for the recovery of NH₃ and CO₂;
~ a stream 8, containing water, OATs and a quantity of NH₃ which is such as to avoid the precipitation of OATs, which ranges from 0.1 to 2% by weight with respect to the total weight of stream 8, sent to the subsequent section (OE) for the elimination of the OATs.

The substantial difference between the process according to the present invention and the state of the art consists in the fact that the distillation is carried out in a distillation column (AR) which operates at a temperature at the bottom not exceeding 185°C, preferably from 150 to 180°C, and even more preferably from 115 to 150°C. The distillation is carried out at a pressure ranging from 0.2 to 1.0 MPa.

### OATs Elimination Section OE:

In the process according to the present invention, as shown in figure 2, a stream 8, comprising water, OATs and a quantity of NH₃ which is such as to avoid the precipitation of OATs, exits from the deammoniation section AR and enters the OATs elimination section OE.

In the process according to the present invention, this stream 8 is treated in order to obtain a stream of pure water 9 and stream 12 in vapour phase, comprising NH₃ and water-saturated CO₂. The stream of pure water 9 is recycled to the quench-ammonolysis section QAL, whereas the stream 12, containing NH₃ and water-saturated CO₂, is sent to the off-gas treatment and then recycled to the Urea plant to recover NH₃ and CO₂ contained therein, or it is sent directly to the urea plant.

The section OE effects a decomposition reaction of OATs carried out at the same pressure as the reaction section R, or at a higher pressure.

The gas stream 12 is composed of NH₃ and CO₂ saturated with water and the lower the water content of stream 12, the higher the operating pressure of the section OE will be, which ranges from 7.0 to 12.0 MPa, more preferably from 8.0 to 11.0 MPa.

Furthermore, by operating at high pressure, the water content of stream 12 is extremely reduced with a consequent benefit in the conversion of NH₃ and CO₂ into urea in the urea plant.

Thanks to the modification in the temperature values in the distillation column in the deammoniation section (AR), the column life has been significantly increased, avoiding fouling phenomena of the column itself and maintaining its efficiency for a long time, thus also ensuring better interaction with the OATs elimination section (OE), and the improvement in the integration of the urea plant with the melamine plant, in terms of the effectiveness of the conversion of the NH₃ and CO₂ reagents into urea: the liquid or gaseous streams fed to the urea synthesis process/plant for the recovery of the NH₃ and CO₂ contained therein do not in fact contain water or contain water in extremely small quantities, thus allowing a significant improvement in the conversion of the NH₃ and CO₂ reagents into urea.

Furthermore, the operating mode of the deammoniation section (AR) allows a stream 8 to be obtained, comprising water, OATs and a quantity of NH₃ which is such as to avoid the precipitation of OATs.

The present invention also further relates to an apparatus for effecting a deammoniation or ammonia recovery step in a process for the production of melamine, said deammoniation or ammonia recovery step being carried out on a stream of mother liquor containing melamine crystals (6) coming from a liquid/solid separation step (SLS) of an aqueous ammonia stream, said apparatus comprising:
i) a deammoniation section (AR) suitable for treating, by means of a distillation column having a temperature at the bottom of the column not exceeding 185°C, a part of the mother liquors (stream 6) coming from the melamine liquid/solid separation section (SLS), said deammoniation section (AR) being connected to said liquid/solid separation section (SLS) from which it receives the mother liquor stream (6) and to a quench-ammonolysis section (QAL) to which it sends a stream containing water and ammonia (10);
ii) said deammoniation section (AR) also being connected to an OATs elimination section (OE) to which it sends a stream (8) containing water, OATs, and a quantity of NH₃ which is such as to avoid the precipitation of OATs, said OATs elimination section (OE) also being connected to the quench-ammonolysis section (QAL) to which it sends a liquid phase (9) substantially composed of water.

### EXAMPLES:

The following table indicates four different comparative cases obtained from tests carried out on the ammonia distillation column (AR) in the plant.

| | **Temperature [°C]** | **Pressure [MPa]** | **[%wt] NH₃** | **pH** | **Concentration of OATs Stream (6) [ppm]** | **OATs Precipitation** |
|---|---|---|---|---|---|---|
| EXAMPLE 1 (comp) | 205 | 1.6 | 0.01% | 9 | 4500 | SI |
| EXAMPLE 2 (comp) | 195 | 1.3 | 0.01% | 9 | 3500 | YES |
| EXAMPLE 3 | 185 | 1.0 | 0.5% | 11.3 | 3500 | NO |
| EXAMPLE 4 | 145 | 0.32 | 1% | 11.5 | 3500 | NO |

Example 1 is representative of the deammoniation process of the state of the art wherein the temperature at the bottom of the column is equal to 205°C and the concentration of ammonia at the bottom of the column is practically negligible (0.01% by weight or 100 ppm). At the inlet to the deammoniation step, the stream (6) contains 4500 ppm of OATs which are situated at the bottom of the column. The example shows that at 205°C, with a concentration of OATs at the inlet equal to 4500 ppm, said quantity of OATs precipitates into a fluid practically almost devoid of NH₃ (pH approximately 9), causing fouling of the plates of the column.

Example 2 is also representative of the deammoniation process of the state of the art wherein the temperature at the bottom of the column is equal to 195°C and the concentration of ammonia at the bottom of the column is practically negligible (0.01% by weight or 100 ppm). At the inlet to the deammoniation step, the stream (6) contains 3500 ppm of OATs which are situated at the bottom of the column. The example shows that at 195°C, with a concentration of OATs at the inlet equal to 3500 ppm, said quantity of OATs precipitates into a fluid practically almost devoid of NH₃ (pH approximately 9), causing fouling of the plates of the column.

Examples 3 and 4 are representative of the present invention.

Operating within the concentration range of ammonia defined for the process according to the present invention (0.5% by weight for Example 3 and 1% by weight for Example 4), the higher pH favours the solubilization of the OATs (3500 ppm) present on the bottom of the column which can thus operate at lower temperatures (Example 3: 185°C and relative equilibrium pressure 1.0 Mpa; Example 4; 145°C and relative equilibrium pressure 0.32 Mpa).

Examples 3 and 4 demonstrate how the combination of the parameters according to the present invention in terms of temperature and NH₃ concentration surprisingly allow the fouling of the plates of the distillation column to be avoided, preventing the precipitation of OATs, and thus favouring the efficiency and operation of the distillation column and the sections downstream of the column.

## Claims

1. A process for the production of high-purity melamine with high yields by the pyrolysis of urea carried out at a temperature ranging from 360 to 450°C and at a pressure higher than 7.0 MPa, comprising a deammoniation step or recovery of ammonia from a stream of mother liquor coming from a liquid/solid separation step (SLS) of an ammonia aqueous stream containing melamine crystals, wherein said mother liquor stream (6) is treated in a deammoniation section (AR) from which the following are obtained by distillation:
~ a stream (10), composed of NH₃ and water, which is recycled to the quench-ammonolysis section (QAL);
~ a stream (11), composed of water, CO₂ and NH₃ which is sent to an off-gas treatment step or recycled directly to a urea plant for the recovery of NH₃ and CO₂;
~ a stream (8), containing water, OATs and a quantity of NH₃ that ranges from 0.1 to 2% by weight with respect to the weight of stream 8, which is sent to the subsequent section (OE) for the elimination of the OATs,
said distillation step being carried out in a distillation column with a temperature at the bottom of the column not exceeding 185°C.

2. The process according to claim 1, wherein the temperature at the bottom of the column ranges from 150 to 180°C.

3. The process according to claim 1, wherein the temperature at the bottom of the column ranges from 115 to 150°C.

4. The process according to claim 1, wherein the distillation step is carried out at a pressure ranging from 0.2 to 1.0 MPa.

5. An apparatus for effecting a deammoniation or ammonia recovery step in a melamine production process, said deammoniation or ammonia recovery step being carried out on a stream of mother liquor containing melamine crystals (6) coming from a liquid/solid separation step (SLS) of an aqueous ammonia stream, said apparatus comprising:
i) a deammoniation section (AR) suitable for treating, by means of a distillation column having a temperature at the bottom of the column not exceeding 185°C, a part of the mother liquors (stream 6) coming from the melamine liquid/solid separation section (SLS), said deammoniation section (AR) being connected to said liquid/solid separation section (SLS) from which it receives the stream of mother liquor (6) and to a quench-ammonolysis section (QAL) to which it sends a stream containing water and ammonia (10);
ii) said deammoniation section (AR) also being connected to an OATs elimination section (OE) to which it sends a stream (8) containing water, OATs, and a quantity of NH₃ which is such as to avoid the precipitation of OATs, said OATs elimination section (OE) also being connected to the quench-ammonolysis section (QAL) to which it sends a liquid phase (9) substantially composed of water.

## Amended claims

### Amended claims in accordance with Rule 137(2) EPC.

1. A process for the production of high-purity melamine with high yields by the pyrolysis of urea carried out at a temperature ranging from 360 to 450°C and at a pressure higher than 7.0 MPa, comprising a deammoniation step or recovery of ammonia from a stream of mother liquor coming from a liquid/solid separation step (SLS) of an ammonia aqueous stream containing melamine crystals, wherein said mother liquor stream (6) is treated in a deammoniation section (AR) from which the following are obtained by distillation:
~ a stream (10), composed of NH₃ and water, which is recycled to the quench-ammonolysis section (QAL);
~ a stream (11), composed of water, CO₂ and NH₃ which is sent to an off-gas treatment step or recycled directly to a urea plant for the recovery of NH₃ and CO₂;
~ a stream (8), containing water, OATs and a quantity of NH₃ that ranges from 0.1 to 2% by weight with respect to the weight of stream 8, which is sent to the subsequent section (OE) for the elimination of the OATs,
said distillation step being carried out in a distillation column with a temperature at the bottom of the column not exceeding 185°C.

2. The process according to claim 1, wherein the temperature at the bottom of the column ranges from 150 to 180°C.

3. The process according to claim 1, wherein the temperature at the bottom of the column ranges from 115 to 150°C.

4. The process according to claim 1, wherein the distillation step is carried out at a pressure ranging from 0.2 to 1.0 MPa.

5. An apparatus for effecting a deammoniation or ammonia recovery step in a melamine production process according to anyone of claims 1-4, said deammoniation or ammonia recovery step being carried out on a stream of mother liquor containing melamine crystals (6) coming from a liquid/solid separation step (SLS) of an aqueous ammonia stream, said apparatus comprising:
i) a deammoniation section (AR) suitable for treating, by means of a distillation column having a temperature at the bottom of the column not exceeding 185°C, a part of the mother liquors (stream 6) coming from the melamine liquid/solid separation section (SLS), said deammoniation section (AR) being connected to said liquid/solid separation section (SLS) from which it receives the stream of mother liquor (6) and to a quench-ammonolysis section (QAL) to which it sends a stream containing water and ammonia (10);
ii) said deammoniation section (AR) also being connected to an OATs elimination section (OE) to which it sends a stream (8) containing water, OATs, and a quantity of NH₃ which is such as to avoid the precipitation of OATs, said OATs elimination section (OE) also being connected to the quench-ammonolysis section (QAL) to which it sends a liquid phase (9) substantially composed of water.
